# FASCICULE DE BREVET EUROPEEN

(11) **EP 1 537 894 B1**
(45) Date de publication et mention de la délivrance du brevet: **28.03.2007**
(21) Numéro de dépôt: 04292864.8
(22) Date de dépôt: 03.12.2004
(51) Int. Cl.: A61N 1/37, A61N 1/365

(54) **Dispositif médical implantable actif à fonctions d'enregistrement Holter**
Implantierbares medizinisches Gerät mit Aufzeichnungsfunktionen von Holter
Implantable medical device with Holter's recording functions

(30) Priorité: 03.12.2003 FR 0314158
(43) Date de publication de la demande: 08.06.2005
(73) Titulaire: ELA MEDICAL, 92541 Montrouge (FR)
(72) Inventeur: Henry, Christine, 75014 Paris (FR); Poezevara, Yann, 91080 Courcouronnes (FR)
(74) Mandataire: Dupuis-Latour, Dominique

(56) Documents cités:
- EP-A- 0 653 226
- US-A1- 2002 173 829
- US-A1- 2002 193 697
- US-B1- 6 200 265
- US-B1- 6 351 670

## Description

L'invention concerne les dispositifs médicaux implantables actifs tels que définis par la directive 90/385/CE du 20 juin 1990 du Conseil des communautés européennes.

L'invention concerne des implants actifs tels que stimulateur cardiaque, cardioverteur et/ou défibrillateur ou dispositif multisite, c'est-à-dire des appareils capables d'appliquer une thérapie antibradycardique et/ou antitachycardique, mais elle peut également à être mise en oeuvre avec des implants dont le seul objet est le suivi et l'enregistrement de paramètres physiologiques, comme celui décrit dans le WO-A-98/02209 (Medtronic Inc.).

L'invention concerne plus précisément ceux de ces dispositifs qui sont dotés de fonctions Holter, c'est-à-dire de fonctions d'enregistrement sur une longue période, pouvant aller de plusieurs jours à plusieurs mois, de données recueillies par le dispositif, typiquement des données relatives à l'activité cardiaque ou ventilatoire du patient. Les informations ainsi enregistrées peuvent être transmises ultérieurement, par télémétrie, à un appareil externe ou "programmateur" pour visualisation et analyse.

Les signaux physiologiques recueillis par ces implants peuvent être de natures très différentes :
- signaux représentatifs de l'activité et du rythme cardiaque mesurés au moyen d'électrodes auriculaires et/ou ventriculaires implantées - ces signaux sont habituellement dénommés "électrogrammes", par opposition aux "électrocardiogrammes" recueillis par des électrodes externes, non implantées ;
- signaux représentatifs de l'activité respiratoire, par exemple signal de ventilation-minute obtenu par mesure de l'impédance transthoracique prise entre le boîtier métallique du dispositif et une électrode distante ;
- signaux représentatifs du débit sanguin ou de la contractilité du myocarde, obtenus à partir d'une mesure d'impédance intracardiaque (impédance transvalvulaire, entre deux cavités situées d'un même côté du coeur, ou impédance transseptale - interventriculaire ou oblique -, prise entre deux cavités situées de deux côtés différents) ;
- signaux de mesure de la saturation en oxygène du sang, de mesure du pH sanguin, etc.

On inclura dans cette notion de "signaux", non seulement les signaux proprement dits recueillis par des électrodes, mais également des marqueurs d'événements du rythme cardiaque, compteurs d'événements ou signaux non recueillis mais représentatifs d'un état ou d'une action de l'implant, par exemple l'application d'une thérapie, une commutation de mode de fonctionnement, etc.

Le EP-A-0 653 226 (ELA Medical) décrit un tel dispositif implantable, en l'espèce un cardioverteur/défibrillateur, pourvu de fonctions d'enregistrement Holter et permettant d'opérer une mémorisation à plusieurs niveaux de détail : en cas d'événement (survenue d'un trouble du rythme, application d'une thérapie, ...), le dispositif enregistre pendant une courte durée des informations plus détaillées (typiquement, un échantillon d'électrogramme de la période ayant précédé et/ou suivi l'événement particulier), tandis qu'il enregistre simplement une chaîne de marqueurs le reste du temps.

Dans ce dispositif connu, comme dans les autres implants intégrant des fonctions d'enregistrement Holter, la quasi-totalité de la mémoire - dont la taille est nécessairement limitée du fait des contraintes de miniaturisation et de consommation - est réservée pour la mémorisation des informations concernant les nombreux algorithmes et fonctions disponibles tels que statistiques, histogrammes, épisodes d'électrogramme, marqueurs déclenchés sur des événements particuliers, etc. C'est d'ailleurs la raison pour laquelle le EP-A-0 653 226 précité propose de ne mémoriser l'électrogramme que sur une très brève durée, limitée aux instants précédant et suivant le déclenchement d'une thérapie, typiquement les deux secondes précédant et suivant le choc de cardioversion ou de défibrillation.

Dans certaines circonstances, il peut être cependant utile de recueillir et mémoriser un volume d'informations important, dédié à une fonction particulière, pendant une longue période donnée, par exemple sur 24 h.

L'un des buts de la présente invention est de proposer un implant pourvu de fonctions d'enregistrement Holter qui puisse être à volonté dédié à l'enregistrement en temps réel et en continu d'une fonction particulière.

Ainsi, par exemple pour l'étude des troubles liés à l'apnée du sommeil, il serait intéressant de pouvoir sauvegarder en continu et sur 24 heures, ou à tout le moins sur une partie de la nuit, l'intégralité du signal de ventilation-minute.

Ou encore, pour les troubles du rythme cardiaque ou l'évaluation de la conduction auriculo-ventriculaire, il serait intéressant de pouvoir sauvegarder en continu la totalité de l'électrogramme sur 24 heures, pour pouvoir ultérieurement l'analyser de la même manière qu'un enregistrement d'électrocardiogramme produit par un appareil externe relié à des électrodes de surface.

L'idée de base de l'invention consiste à prévoir un dispositif implantable dont la mémoire Holter puisse être utilisée soit d'une manière standard (c'est-à-dire en mémorisant les données habituelles telles que statistiques, histogrammes, marqueurs, etc.), soit de manière dédiée au suivi d'une fonction particulière.

Dans la configuration Holter dédiée, l'appareil consacre la majeure partie de sa mémoire à l'enregistrement en continu du signal choisi, conservant seulement quelques octets pour des informations générales sur les autres fonctions de l'appareil.

Le dispositif implanté de l'invention non seulement sauvegarde en continu le signal suivi mais, en outre, analyse en temps réel ce signal.

Dans l'exemple où le signal suivi est un signal ventilatoire, cette analyse permet d'identifier certains événements tels qu'apnée, hypopnée ou pause respiratoire, ou des profils respiratoires particuliers, tels que des profils de type Cheyne-Stokes. On pourra se référer à cet égard aux EP-A-0 970 713, EP-A-1 336 422 et EP-A-1 295 623, tous trois au nom d'ELA Médical.

Dans l'exemple où le signal suivi est un signal cardiaque, l'analyse permet d'identifier des événements tels qu'une arythmie ou un trouble de la conduction.

La détection de ces événements provoque alors le déclenchement de la mémorisation d'une ou plusieurs "informations de contexte", c'est-à-dire d'informations parallèles, distinctes du signal suivi, qui permettront d'étudier ultérieurement le contexte de survenue des événements, notamment à des fins de diagnostic.

Par exemple, si le signal suivi est le signal ventilatoire, les informations de contexte pourront être un épisode d'électrogramme, un signal de pic d'accélération endocardiaque, un signal délivré par un capteur d'activité du patient tel qu'un accéléromètre intégré au boîtier du dispositif, ou tout autre information permettant de documenter l'événement (apnée, hypopnée, ...) détecté sur le signal suivi.

Plus précisément, la présente invention propose un dispositif médical implantable actif à fonctions d'enregistrement Holter du type décrit par le EP-A-0 653 226 précité, c'est-à-dire comprenant les moyens énoncés au préambule de la revendication 1. Selon l'invention, ce dispositif comprend les moyens énoncés dans la partie caractérisante de cette revendication 1. Les diverses sous-revendications visent des formes de mise en oeuvre avantageuses.

On va maintenant décrire plus en détail l'invention, qui peut être mise en oeuvre par une programmation appropriée du logiciel de commande d'un dispositif d'un type en lui-même connu, par exemple de type stimulateur cardiaque ou défibrillateur/cardioverteur, comprenant des moyens d'acquisition d'un signal fourni par des sondes endocavitaires et/ou un ou plusieurs capteurs implantés.

L'invention peut notamment être appliquée aux dispositifs commercialisé par ELA Médical, Montrouge, France. Il s'agit de dispositifs à microprocesseur comportant des circuits pour recevoir, mettre en forme et traiter des signaux électriques recueillis par des électrodes implantées. Ils est possible d'y transmettre par télémétrie des logiciels qui seront conservés en mémoire et exécutés pour mettre en oeuvre les fonctions de l'invention qui seront décrites ci-dessous.

Les différents signaux fournis par les sondes endocavitaires et/ou les capteurs implantés peuvent être notamment :
- des signaux d'électrogramme ventriculaire et/ou auriculaire ;
- des marqueurs d'événements du rythme cardiaque, produit par un algorithme d'analyse interne au dispositif;
- un signal de pic d'accélération endocardiaque, au moyen d'un capteur tel que décrit par exemple dans le EP-A-0 515 319 (Sorin Biomedica Cardio Spa) ;
- un signal représentatif de l'activité respiratoire du patient, typiquement un signal de ventilation-minute obtenu par mesure de l'impédance transthoracique entre le boîtier du stimulateur et une électrode endocavitaire ;
- un signal représentatif du débit sanguin, typiquement obtenu par une mesure de bioimpédance transvalvulaire (entre oreillette et ventricule situé d'un même côté du coeur), transseptale oblique (entre un ventricule et l'oreillette située du côté opposé) ou transseptal interventriculaire (entre les deux ventricules), donnant une valeur corrélée au débit cardiaque et à la contractilité du myocarde ; ou encore
- un signal délivré par un capteur d'activité du patient tel qu'un accéléromètre intégré au boîtier du dispositif.

De façon caractéristique, le dispositif comprend des moyens pour programmer la mémorisation Holter :
- soit selon un mode "standard", c'est-à-dire pour une simple mémorisation de statistiques, histogrammes, marqueurs, compteurs d'événements, brefs épisodes d'électrogrammes sur apparition d'un événement particulier, etc., et ce sur une très longue durée, pouvant aller de plusieurs jours à plusieurs mois, et concernant toutes les fonctions disponibles du dispositif implanté (prévention des fibrillations auriculaires, repli, conduction auriculo-ventriculaire, etc.) ;
- soit selon un mode dit "dédié" spécifique de l'invention. En mode dédié, la mémoire Holter sera utilisée pour mémoriser en continu un signal particulier pendant une durée prédéterminée ; ce signal particulier sera non seulement mémorisé en continu pendant la période considérée, mais également analysé en temps réel par le dispositif pour y détecter la survenue éventuelle d'événements prédéfinis, et sera pour cette raison désigné "signal suivi".

Le signal suivi peut être par exemple :
- le signal de ventilation-minute, si l'on veut dédier l'appareil à l'analyse d'un trouble respiratoire (apnée du sommeil, hypopnée) ;
- l'électrogramme, si l'on veut dédier temporairement l'appareil à l'analyse d'un trouble du rythme cardiaque ou de la conduction auriculo-ventriculaire.

Le signal suivi sera intégralement stocké dans la mémoire Holter sur une durée de 24 heures par exemple, durée qui peut être une période continue de 24 heures, ou une période fractionnée, par exemple- en 6 sous-périodes de 6 heures chacune, répétées 4 fois pendant 4 nuits consécutives.

Le passage du mode standard au mode dédié, et donc le déclenchement du stockage du signal suivi, est réalisé soit à volonté, par une commande envoyée par télémétrie au dispositif par le patient ou par un praticien, soit de façon automatique sur détection de certaines conditions.

Ce passage automatique au mode dédié peut être par exemple déclenché par:
- un signal indicateur d'un début de phase de sommeil du patient, issu d'une horloge interne commutant un indicateur à heure fixe, ou bien obtenu par analyse du signal physiologique de ventilation-minute pour discriminer entre éveil et sommeil, comme cela est enseigné par le EP-A-0 719 568 (ELA Médical), ou
- un signal indicateur de franchissement d'un seuil, par exemple lorsque la fréquence cardiaque dépasse une limite supérieure prédéterminée.

Comme on l'a évoqué plus haut, le signal suivi est analysé en temps réel par le dispositif afin d'y détecter l'éventuelle survenue d'événements tels que :
- apnée ou hypopnée, pour un signal ventilatoire,
- modification de morphologies sur l'électrogramme, pour un signal cardiaque endocavitaire,
- arythmie, pour des marqueurs ou à partir de l'électrogramme,
- changement d'amplitude moyenne, pour un signal d'accélération endocardiaque ou de bioimpédance intracardiaque,
- détection d'un profil répétitif particulier de variation du signal respiratoire, par exemple un profil de type Cheyne-Stokes, comme cela est décrit par exemple dans le EP-A-1 295 623 (ELA Médical) précité,
- baisse de l'amplitude du pic d'accélération endocardiaque,
- etc.

Sur détection d'un événement, le dispositif déclenche l'enregistrement parallèle, sur une brève durée, d'informations additionnelles de contexte. La détection de ces événements peut également servir à déclencher le passage automatique en mode dédié.

Les informations additionnelles de contexte sont par exemple :
- un épisode d'électrogramme et/ou d'accélération endocavitaire et/ou des marqueurs d'événements, sur détection d'une apnée à partir du signal ventilatoire,
- enregistrement de marqueurs et/ou des valeurs de pic d'accélération endocavitaire et/ou d'un épisode de signal ventilatoire, sur détection d'une arythmie à partir de l'électrogramme.

La durée d'enregistrement des informations de contexte est par exemple de 10 secondes, durée en relation avec l'instant de survenue de l'événement détecté.

Le signal suivi enregistré dans la mémoire Holter, de même que les informations de contexte éventuellement enregistrées sur détection d'un événement particulier, sont ultérieurement transmises à un programmateur externe, par télémétrie, afin d'y être visualisées et traitées en temps différé.

Le praticien pourra ainsi accéder simultanément, au moyen du programmateur, aux informations suivantes :
- une courbe sur 24 heures (c'est-à-dire la période de mémorisation du signal suivi) représentant un condensé du signal mémorisé, courbe sur laquelle peuvent être repérés, manuellement ou automatiquement, les événements détectés liés au signal, par exemple les apnées et hypopnées si le signal mémorisé est le signal ventilatoire,
- un "zoom" du signal lorsqu'un événement est repéré, ou bien n'importe où sur la courbe du signal suivi, ainsi que, le cas échéant, les informations de contexte qui ont pu être mémorisées sur détection d'un événement particulier.

Le traitement en différé permet d'appliquer au signal suivi, qui a été mémorisé en continu, des traitements supplémentaires complexes permettant d'extraire des informations qu'il n'aurait pas été possible d'obtenir en temps réel au moyen de l'implant : analyse de Fourier, variabilité sinusale, simulation d'algorithmes de détection d'événements, etc.

Avantageusement, après que les informations stockées dans la mémoire Holter en mode dédié ont été télétransmises au programmateur, ce dernier commande la libération de la mémoire (car il n'est plus nécessaire de conserver les informations dans l'implant une fois que celles-ci ont été transmises et stockées à l'extérieur) puis le basculement automatique du dispositif en mode de fonctionnement standard.

Enfin, on a décrit plus haut le processus d'enregistrement des données opéré sur une durée prédéterminée. Mais d'autres mises en oeuvre sont envisageables en variante, par exemple un enregistrement du signal suivi et/ou des informations de contexte sur une durée allant jusqu'à saturation de la mémoire de données, ou encore sur une durée indéterminée, les données nouvelles étant enregistrées dans la mémoire de données jusqu'à ce que celle-ci soit remplie et les nouvelles données remplaçant ensuite les données plus anciennes, suivant la technique bien connue "premier entré, premier sorti".

## Revendications

1. Un dispositif médical implantable actif à fonctions d'enregistrement Holter, notamment un stimulateur cardiaque, un défibrillateur/cardioverteur ou un dispositif multisite, comprenant :
- des moyens aptes à recueillir des données relatives à l'état du patient porteur du dispositif et/ou du fonctionnement de ce dispositif, et à analyser ces données pour produire en réponse des informations générales représentatives,
- des moyens de recueil en continu d'au moins un signal suivi, représentatif d'une fonction physiologique du patient,
- une mémoire de données, où sont mémorisées de façon durable lesdites informations générales,
- des moyens d'analyse en temps réel du signal suivi, pour y détecter la survenue d'au moins un événement particulier,
- des moyens de gestion mémoire, opérant en réponse aux moyens d'analyse pour scinder la mémoire, sur détection de la survenue d'au moins un événement particulier, en :
· une première zone, pour la mémorisation desdites informations générales et
· une deuxième zone, pour la mémorisation en continu dudit signal suivi ; et
- des moyens aptes, sur détection de la survenue dudit événement particulier par les moyens d'analyse, à dédencher ladite mémorisation en continu du signal suivi dans la deuxième zone de la mémoire pendant une première période de temps,
dispositif **caractérisé en ce qu'**il comprend en outre des moyens d'allocation dynamique de la deuxième zone, aptes à commuter le dispositif entre :
· une configuration standard, où la deuxième zone est allouée à la mémorisation d'une pluralité des dites informations générales en complément de la première zone, et
· une configuration dédiée, où la deuxième zone est allouée à la mémorisation en continu du signal suivi.

2. Le dispositif médical implantable actif de la revendication 1, comprenant en outre des moyens déclencheurs aptes, sur réception d'une commande, à commuter le dispositif en configuration dédiée et commencer la mémorisation en continu du signal suivi dans la deuxième zone.

3. Le dispositif médical implantable actif de la revendication 2, dans lequel ladite commande est: un signal indicateur d'un début de phase de sommeil du patient ; un signal indicateur de franchissement d'un seuil prédéfini par la fréquence cardiaque ; une commande d'origine externe transmise au dispositif par le patient ou un praticien ; et/ou une commande interne préprogrammée générée par le dispositif.

4. Le dispositif médical implantable actif de la revendication 1, dans lequel:
- le dispositif comprend en outre des moyens de recueil d'informations de contexte,
- les moyens de gestion mémoire opèrent en réponse aux moyens d'analyse pour scinder la mémoire, outre la première zone et la deuxième zone, en :
· une troisième zone, pour la mémorisation conditionnelle desdites informations de contexte, et
- le dispositif comprend également des moyens aptes, sur détection de la survenue dudit événement particulier par les moyens d'analyse, à déclencher ladite mémorisation des informations de contexte dans la troisième zone de la mémoire, pendant une deuxième période de temps plus brève que ladite première période de mémorisation du signal suivi.

5. Le dispositif médical implantable actif de la revendication 1, dans lequel ledit signal suivi représentatif d'une fonction physiologique du patient est un signal ventilatoire.

6. Le dispositif médical implantable actif de la revendication 5, dans lequel ledit événement particulier dont la survenue est détectée par les moyens d'analyse est une apnée et/ou une hypopnée.

7. Le dispositif médical implantable actif de la revendication 5, dans lequel ledit événement particulier dont la survenue est détectée par les moyens d'analyse est un motif prédéterminé répétitif du signal respiratoire.

8. Le dispositif médical implantable actif des revendications 4 et 5 prises en combinaison, dans lequel lesdites informations de contexte comprennent : un signal d'électrogramme ventriculaire et/ou auriculaire ; des marqueurs d'événements du rythme cardiaque ; et/ou un signal de pic d'accélération endocardiaque.

9. Le dispositif médical implantable actif de la revendication 1, dans lequel le signal suivi représentatif d'une fonction physiologique du patient, est : un signal d'électrogramme ventriculaire et/ou auriculaire ; un signal de pic d'accélération endocardiaque ; une série de marqueurs d'événements du rythme cardiaque ; et/ou un signal de bioimpédance intracardiaque.

10. Le dispositif médical implantable actif de la revendication 9, dans lequel ledit événement particulier dont la survenue est détectée par les moyens d'analyse est un trouble prédéfini du rythme cardiaque et/ou un trouble de la conduction auriculo-ventriculaire.

11. Le dispositif médical implantable actif des revendications 4 et 9 prises en combinaison, dans lequel lesdites informations de contexte comprennent : des marqueurs d'événements du rythme cardiaque ; un signal de pic d'accélération endocardiaque ; et/ou un signal ventilatoire.

12. Le dispositif médical implantable actif de la revendication 1 ou 4, comprenant en outre des moyens de télétransmission à un programmateur externe du contenu de la deuxième, ainsi que du contenu de la troisième zone s'il y a été mémorisé des informations de contexte, pour visualisation et analyse en temps différé de ce(s) contenu(s) par le programmateur.

13. Le dispositif médical implantable actif de la revendication 12, comprenant en outre des moyens aptes à libérer la deuxième et la troisième zone après télétransmission de leur contenu, pour y autoriser la mémorisation ultérieure de nouvelles données.

14. Le dispositif médical implantable actif des revendications 2 et 13 prises en combinaison, dans lequel les moyens aptes à libérer la deuxième et la troisième zone après télétransmission sont également aptes à recommuter le dispositif en configuration standard après télétransmission.

15. Le dispositif médical implantable actif de la revendication 1 ou de la revendication 4, dans lequel ladite première et/ou ladite deuxième période de temps sont des périodes de temps prédéterminées.

16. Le dispositif médical implantable actif de la revendication 15, dans lequel ladite première période de temps est une période continue unique.

17. Le dispositif médical implantable actif de la revendication 15, dans lequel ladite première période de temps est une période fractionnée en une pluralité de sous-périodes quotidiennes.

18. Le dispositif médical implantable actif de la revendication 15, dans lequel ladite première période de temps est une période de 24 heures.

19. Le dispositif médical implantable actif de la revendication 15, dans lequel ladite deuxième période de temps est une période en relation avec l'instant de survenue dudit événement particulier.

20. Le dispositif médical implantable actif de la revendication 19, dans lequel ladite deuxième période de temps est une période de 10 secondes.

21. Le dispositif médical implantable actif de la revendication 1 ou de la revendication 4, dans lequel ladite première et/ou ladite deuxième période de temps sont des périodes de temps durant jusqu'à saturation de la mémoire de données.

22. Le dispositif médical implantable actif de la revendication 1 ou de la revendication 4, dans lequel ladite première et/ou ladite deuxième période de temps sont des périodes de temps indéterminées, les données nouvelles étant enregistrées dans la mémoire de données jusqu'à ce que celle-ci soit remplie et les nouvelles données remplaçant ensuite les données plus anciennes.

## Claims

1. An active implantable medical device with Holter recording functions, such as a cardiac pacemaker, defibrillator/cardioverter or multisite device, comprising:
- means able to collect data relating to the state of the patient carrying the device and/or operation of this device, and to analyze this data to produce in response general representative information,
- means for continuous collection of at least one monitored signal representative of a physiological function of the patient,
- a data memory, where said general information is memorized in a durable way,
- means for analyzing the monitored signal in real time to detect therein the occurrence of at least one particular event,
- memory management means, operating in response to the analyzing means in order to split the memory, on detection of the occurrence of at least one particular event, in:
· a first zone for memorizing said general information, and
· a second zone for continuous memorizing of said monitored signal; and
- means able, on detection of the occurrence of said particular event by the analyzing means, to trigger said continuous memorizing of the monitored signal in the second memory zone over a first time period,
the device being **characterised in that** it further comprises means for dynamic allocation of the second zone, able to commutate the device between:
- a standard configuration, wherein the second zone is allocated to memorizing a plurality of general information elements in complement to the first zone, and
- a dedicated configuration, wherein the second zone is allocated to continuous memorizing of the monitored signal.

2. The active implantable medical device of claim 1, further comprising triggering means able, on reception of a command, to commutate the device to the dedicated configuration and to begin to continuously memorize the monitored signal in the second zone.

3. The active implantable medical device of claim 2, wherein said command is: a signal indicating a beginning of a sleep phase of the patient; a signal indicating a crossing of a predefined threshold by the heart rate; a command of external origin transmitted to the device by the patient or the practitioner; and/or a pre-programmed internal command generated by the device.

4. The active implantable medical device of claim 1, wherein:
- the device further comprises means for collecting context information,
- the memory management means operate in response to the analyzing means to split the memory, in addition to the first zone and the second zone, into:
· a third zone, for conditional memorizing of said context information, and
- wherein the device equally comprises means able, on detection of the occurrence of said particular event by the analyzing means, to trigger said memorizing of the context information in the third memory zone over a second time period shorter than the said first period for memorising the monitored signal.

5. The active implantable medical device of claim 1, wherein said monitored signal representative of a physiological function of the patient is a ventilatory signal.

6. The active implantable medical device of claim 5, wherein said particular event, the occurrence of which is detected by the analyzing means, is an apnea and/or a hypopnea.

7. The active implantable medical device of claim 5, wherein said particular event, the occurrence of which is detected by the analyzing means, is a repetitive predetermined motif of the respiratory signal.

8. The active implantable medical device of claims 4 and 5 in combination, wherein said context information comprises: a ventricular and/or atrial electrogram signal; event markers of the heart rate; and/or a peak endocardial acceleration signal.

9. The active implantable medical device of claim 1, wherein the monitored signal representative of a physiological function of the patient is: a ventricular and/or atrial electrogram signal; a peak endocardial acceleration signal; a series of event markers of the heart rate; and/or an intracardiac bio-impedance signal.

10. The active implantable medical device of claim 9, wherein said particular event, the occurrence of which is detected by the analyzing means, is a predefined heart rate disorder and/or a disorder of the atrio-ventricular conduction.

11. The active implantable medical device of claims 4 and 9 in combination, wherein said context information comprises: event markers of the heart rate; a peak endocardial acceleration signal; and/or a ventilatory signal.

12. The active implantable medical device of claim 1 or 4, further comprising means for the teletransmission of the content of the second, as well as the content of the third zone, if context information has been memorised therein, to an external programmer for display and time-delayed analysis of this(these) content(s) by the programmer.

13. The active implantable medical device of claim 12, further comprising means able to free the second and third zone after teletransmission of their content, for authorizing the later memorisation of new data therein.

14. The active implantable medical device of claims 2 and 13 in combination, wherein the means able to free the second and third zone after teletransmission are equally able to recommutate the device into the standard configuration after teletransmission.

15. The active implantable medical device of claim 1 or claim 4, wherein said first and/or said second time period are predetermined time periods.

16. The active implantable medical device of claim 15, wherein said first time period is a unitary continuous period.

17. The active implantable medical device of claim 15, wherein said first time period is a period split into a plurality of daily sub-periods.

18. The active implantable medical device of claim 15, wherein said first time period is a 24 hour period.

19. The active implantable medical device of claim 15, wherein said second time period is a period in relation to the moment of occurrence of said particular event.

20. The active implantable medical device of claim 19, wherein said second time period is a 10 second period.

21. The active implantable medical device of claim 1 or claim 4, wherein said first and/or second time period are time periods that last until saturation of the data memory.

22. The active implantable medical device of claim 1 or claim 4, wherein said first and/or said second time period are undetermined time periods, the new data being recorded in the data memory until filled, and the new data then replacing the older data.

## Patentansprüche

1. Aktive implantierbare medizinische Vorrichtung mit Holter-Speicherfunktionen, insbesondere Herzschrittmacher, Defibrillator/Kardioverter oder Multisite-Vorrichtung, mit:
- Mitteln, die geeignet sind, Daten bezüglich des Zustands des die Vorrichtung tragenden Patienten und/oder des Betriebs dieser Vorrichtung aufzunehmen, und diese Daten zu analysieren, um in Antwort darauf allgemeine repräsentative Informationen zu erzeugen,
- Mitteln zur fortwährenden Aufnahme wenigstens eines verfolgten Signals, das für eine physiologische Funktion des Patienten repräsentativ ist,
- einem Datenspeicher, in welchem die allgemeinen Informationen dauerhaft gespeichert werden,
- Mitteln zur Echtzeitanalyse des verfolgten Signals, um in diesem das Auftreten wenigstens eines besonderen Ereignisses zu erfassen,
- Mitteln zur Speicherverwaltung, die in Antwort auf die Analysemittel arbeiten, um den Speicher bei Erfassung des Auftretens wenigstens eines besonderen Ereignisses in:
· eine erste Zone zur Speicherung der allgemeinen Informationen und
· eine zweite Zone zur fortwährenden Speicherung des verfolgten Signals
aufzuteilen; und
- Mitteln, die geeignet sind, bei Erfassung des Auftretens des besonderen Ereignisses durch die Analysemittel die fortwährende Speicherung des verfolgten Signals in der zweiten Zone des Speichers über einen ersten Zeitabschnitt auszulösen,
wobei die Vorrichtung **dadurch gekennzeichnet ist, dass** sie außerdem Mittel zur dynamischen Zuweisung der zweiten Zone umfasst, die geeignet sind, die Vorrichtung zwischen:
- einer Standardkonfiguration, bei welcher die zweite Zone in Ergänzung der ersten Zone der Speicherung einer Mehrzahl der allgemeinen Information zugewiesen ist, und
- einer eigens vorgesehenen Konfiguration, bei welcher die zweite Zone der fortwährenden Speicherung des verfolgten Signals zugewiesen ist,
umzuschalten.

2. Aktive implantierbare medizinische Vorrichtung nach Anspruch 1, weiterhin mit Auslösemitteln, die geeignet sind, bei Empfang eines Befehls die Vorrichtung in die eigens vorgesehene Konfiguration umzuschalten und die fortwährende Speicherung des verfolgten Signals in der zweiten Zone zu beginnen.

3. Aktive implantierbare medizinische Vorrichtung nach Anspruch 2, wobei der Befehl folgendes ist: ein Signal, das den Beginn einer Schlafphase des Patienten angibt; ein Signal, das das Durchqueren einer vorbestimmten Schwelle durch die Herzfrequenz angibt; ein von außen kommender Befehl, der durch den Patienten oder einen Arzt an die Vorrichtung übertragen wird; und/oder ein vorprogrammierter interner Befehl, der durch die Vorrichtung erzeugt wird.

4. Aktive implantierbare medizinische Vorrichtung nach Anspruch 1, bei welcher:
- die Vorrichtung außerdem Mittel zur Aufnahme von Kontextinformationen umfasst,
- die Speicherverwaltungsmittel in Antwort auf die Analysemittel arbeiten, um den Speicher zusätzlich zur ersten Zone und zweiten Zone in:
· eine dritte Zone zur bedingten Speicherung der Kontextinformationen aufzuteilen, und
- die Vorrichtung ebenso Mittel umfasst, die geeignet sind, bei Erfassung des Auftretens des besonderen Ereignisses durch die Analysemittel die Speicherung der Kontextinformationen in der dritten Speicherzone über einen zweiten Zeitabschnitt auszulösen, der kürzer als der erste Zeitabschnitt der Speicherung des verfolgten Signals ist.

5. Aktive implantierbare medizinische Vorrichtung nach Anspruch 1, bei welcher das verfolgte Signal, das für eine physiologische Funktion des Patienten repräsentativ ist, ein Ventilationssignal ist.

6. Aktive implantierbare medizinische Vorrichtung nach Anspruch 5, bei welcher das besondere Ereignis, dessen Auftreten durch die Analysemittel erfasst wird, eine Apnoe und/oder eine Hypopnoe ist.

7. Aktive implantierbare medizinische Vorrichtung nach Anspruch 5, bei welcher das besondere Ereignis, dessen Auftreten durch die Analysemittel erfasst wird, ein sich wiederholendes vorbestimmtes Motiv des Atemsignals ist.

8. Aktive implantierbare medizinische Vorrichtung der Ansprüche 4 und 5 zusammen genommen, bei welcher die Kontextinformationen folgendes umfassen: ein Signal eines ventrikulären und/oder atrialen Elektrogramms; Marker von Ereignissen des Herzrhythmus; und/oder ein Signal der Spitze einer endokardialen Beschleunigung.

9. Aktive implantierbare medizinische Vorrichtung nach Anspruch 1, bei welcher das verfolgte Signal, das für eine physiologische Funktion des Patienten repräsentativ ist, folgendes ist: ein Signal eines ventrikulären und/oder atrialen Elektrogramms; ein Signal der Spitze einer endokardialen Beschleunigung; eine Serie von Markern von Ereignissen des Herzrhythmus; und/oder ein Signal intrakardialer Bioimpedanz.

10. Aktive implantierbare medizinische Vorrichtung nach Anspruch 9, bei welcher das besondere Ereignis, dessen Auftreten durch die Analysemittel erfasst wird, eine vordefinierte Herzrhythmusstörung und/oder eine Störung der atrial-ventrikulären Leitung ist.

11. Aktive implantierbare medizinische Vorrichtung der Ansprüche 4 und 9 zusammen genommen, bei welcher die Kontextinformationen folgendes umfassen: Marker von Ereignissen des Herzrhythmus; ein Signal der Spitze einer endokardialen Beschleunigung; und/oder ein Ventilationssignal.

12. Aktive implantierbare medizinische Vorrichtung nach Anspruch 1 oder 4, weiterhin mit Mitteln zur Fernübertragung des Inhalts der zweiten, sowie des Inhalts der dritten Zone, falls dort Kontextinformationen gespeichert wurden, an eine externe Programmiereinrichtung zur Visualisierung und zeitversetzten Analyse dieses(dieser) Inhalts(Inhalte) durch die Programmiereinrichtung.

13. Aktive implantierbare medizinische Vorrichtung nach Anspruch 12, weiterhin mit Mitteln, die geeignet sind, die zweite und die dritte Zone nach Fernübertragung deren Inhalts freizugeben, um dort die spätere Speicherung neuer Daten zu erlauben.

14. Aktive implantierbare medizinische Vorrichtung der Ansprüche 2 und 13 zusammen genommen, bei welcher die Mittel, die geeignet sind, die zweite und die dritte Zone nach der Fernübertragung freizugeben, ebenfalls geeignet sind, die Vorrichtung nach der Fernübertragung in die Standardkonfiguration zurückzuschalten.

15. Aktive implantierbare medizinische Vorrichtung nach Anspruch 1 oder nach Anspruch 4, bei welcher der erste und/oder der zweite Zeitabschnitt vorbestimmte Zeitabschnitte sind.

16. Aktive implantierbare medizinische Vorrichtung nach Anspruch 15, bei welcher der erste Zeitabschnitt ein zusammenhängender ununterbrochener Abschnitt ist.

17. Aktive implantierbare medizinische Vorrichtung nach Anspruch 15, bei welcher der erste Zeitabschnitt ein Abschnitt ist, der in eine Mehrzahl von täglichen Unterabschnitten unterteilt ist.

18. Aktive implantierbare medizinische Vorrichtung nach Anspruch 15, bei welcher der erste Zeitabschnitt ein 24-Stunden-Abschnitt ist.

19. Aktive implantierbare medizinische Vorrichtung nach Anspruch 15, bei welcher der zweite Zeitabschnitt ein Abschnitt ist, der in Beziehung zum Zeitpunkt des Auftretens des besonderen Ereignisses steht.

20. Aktive implantierbare medizinische Vorrichtung nach Anspruch 19, bei welcher der zweite Zeitabschnitt ein Abschnitt von 10 Sekunden ist.

21. Aktive implantierbare medizinische Vorrichtung nach Anspruch 1 oder Anspruch 4, bei welcher der erste und/oder der zweite Zeitabschnitt Zeitabschnitte sind, die bis zur Sättigung des Datenspeichers andauern.

22. Aktive implantierbare medizinische Vorrichtung nach Anspruch 1 oder Anspruch 4, bei welcher der erste und/oder der zweite Zeitabschnitt unbestimmte Zeitabschnitte sind, wobei die neuen Daten im Datenspeicher gespeichert werden, bis dieser gefüllt ist, und wobei die neuen Daten anschließend die älteren Daten ersetzen.
